# DEMANDE DE BREVET EUROPEEN

(11) **EP 2 111 819 A1**
(43) Date de publication de la demande: **28.10.2009**
(21) Numéro de dépôt: 08156587.1
(22) Date de dépôt: 05.11.2003
(51) Int. Cl.: A61F 2/08

(54) **Matériel de réparation d'un tissu biologique, notamment d'un tendon ou d'un ligament, et en particulier du tendon calcanéen**

(30) Priorité: 07.11.2002 FR 0213955
(62) Demande divisionnaire de: 03767909.9
(71) Demandeur: Small Bone Innovations International, 01960 Peronnas (FR)
(72) Inventeur: Delponte, Patrick, 29215, Guipavas (FR); Martin, Jean-Jacques, 01000, Bourg-en-bresse (FR)
(74) Mandataire: Jeannet, Olivier

(57) **Abrégé**

Ce matériel comprend un lien (3) et des organes (5) d'arrêt du lien (3) par rapport aux fragments de tissu à réparer.

Selon l'invention,
- l'un des organes d'arrêt est constitué par une vis osseuse (2) pouvant être implantée dans un os environnant le tissu à réparer, notamment dans le calcanéum pour la réparation du tendon calcanéen ;
- le lien (3) forme une partie tubulaire (6) propre à recevoir étroitement une partie de la vis (2) en elle ainsi qu'au moins deux cordons (4) insérables au travers des fragments du tissu biologique à réparer, notamment les deux fragments d'un tendon calcanéen rompu, et
- d'autres organes d'arrêt sont constitués par au moins deux boutons d'arrêt (5).

## Description

La présente invention se rapporte à un matériel de réparation d'un tissu biologique, notamment d'un tendon ou d'un ligament, et en particulier du tendon calcanéen.

Il est connu de réparer un tendon rompu au moyen d'un matériel comprenant :
- un lien relié, à une extrémité, à une aiguille et comprenant un harpon du côté de son autre extrémité,
- une rondelle d'appui, et
- un manchon déformable pouvant être serti sur le lien de manière à immobiliser la rondelle d'appui dans une position déterminée par rapport à ce lien.

L'aiguille permet l'engagement du lien au travers des fragments de tendon, dans le sens longitudinal de ceux-ci, jusqu'à venue du harpon en prise avec le fragment proximal, puis la rondelle et le manchon sont engagés sur l'aiguille et sur le lien. Après mise en tension adéquate du lien, la rondelle est plaquée contre la peau du patient, au niveau de la facette malléolaire, et le manchon est serti sur le lien pour immobiliser ce lien par rapport à la rondelle. La portion du lien dépassant au-delà du manchon est ensuite coupée.

En pratique, deux matériels sont mis en place en parallèle, pour parfaitement caler les deux fragments de tendon l'un par rapport à l'autre. Après le délai de quatre à cinq semaines nécessaire à la cicatrisation des deux fragments de tendon, le lien est sectionné en retrait de la rondelle et est retiré par coulissement.

Ce matériel, et la technique qu'il permet de mettre en oeuvre, donnent satisfaction en pratique. Il apparaît toutefois que les suites de l'intervention restent contraignantes pour le patient et pour le praticien.

En effet, dans le cas de la réparation du tendon calcanéen, la remise en appui du pied du patient ne peut être faite immédiatement et la rééducation ne peut intervenir qu'après un intervalle de temps minimal, ce qui conduit à une déambulation plus ou moins périlleuse du patient pendant cet intervalle de temps. La reprise des activités du patient, notamment d'un entraînement dans le cas d'un sportif, en est différée d'autant. Le risque de rupture itérative existe même si le pourcentage de rupture est reconnu comme acceptable.

Le matériel doit de plus être retiré après la cicatrisation des fragments, ce qui implique une nouvelle intervention. Ce matériel ne peut être laissé en place au-delà de la durée de quatre à cinq semaines précitée, et son ablation relativement précoce n'est pas particulièrement tranquilisante pour le patient ni pour le praticien. Il en résulte que la reprise des activités du patient doit se faire de manière très prudente.

La présente invention vise à remédier à ces inconvénients pratiques importants.

Le matériel qu'elle concerne comprend, de manière connue en soi,
- un lien dont des parties peuvent être insérées au travers des fragments du tissu biologique à réparer, et
- des organes d'arrêt du lien par rapport à ces fragments de tissu.

Selon l'invention,
- l'un des organes d'arrêt est constitué par une vis osseuse pouvant être implantée dans un os environnant le tissu à réparer, notamment dans le calcanéum pour la réparation du tendon calcanéen ;
- le lien forme une partie tubulaire propre à recevoir étroitement une partie de la vis en elle ainsi qu'au moins deux cordons insérables au travers des fragments du tissu biologique à réparer, notamment les deux fragments d'un tendon calcanéen rompu, et
- d'autres organes d'arrêt sont constitués par au moins deux boutons d'arrêt.

La vis est ainsi engagée dans ladite partie tubulaire du lien puis est vissée dans l'os de manière à réaliser l'ancrage d'une extrémité de ce lien par rapport à cet os. Chaque cordon est ensuite inséré au travers des fragments de tissu à réunir puis, après mise en tension adéquate, est solidarisé à un bouton d'arrêt de manière à maintenir les fragments de tissu dans un état de coaptation.

Ce matériel permet d'améliorer de manière significative les suites de l'intervention et le confort du patient en ce sens qu'il permet une rééducation plus précoce, avec, dans le cas d'un tendon calcanéen, mise en appui immédiate du pied du patient, ce qui autorise une déambulation moins périlleuse du patient.

De préférence, la vis, et/ou le lien et/ou les boutons d'arrêt sont en matériau biorésorbable.

Le matériel reste alors en place dans l'organisme jusqu'à résorption, et n'implique par une nouvelle intervention pour son ablation.

Le matériel est efficace pendant une durée, de l'ordre de trois mois, plus longue que la durée d'implantation d'un matériel selon la technique antérieure, ce qui permet une reprise d'activité du patient, notamment d'un entraînement dans le cas d'un sportif, plus précoce et plus sûre pour le patient, et plus tranquilisante pour le chirurgien.

La vis est de préférence une vis dite "d'interférence", c'est-à-dire dépourvue de tête et manoeuvrée au moyen d'une cavité prismatique débouchant dans son extrémité proximale.

La partie tubulaire du lien peut notamment être réalisée par tricotage de fils sous la forme d'une chaussette, et les cordons peuvent être réalisés par tressage de ces mêmes fils.

De préférence, le lien comprend quatre cordons pour permettre une parfaite stabilisation des fragments du tissu biologique à réparer, en particulier le parfait calage d'un fragment de tendon calcanéen par rapport à l'autre.

Chaque cordon pourrait être raccordé de manière permanente à une aiguille au niveau de son extrémité libre, notamment par sertissage de l'extrémité proximale de l'aiguille sur l'extrémité libre du cordon. Chaque cordon est toutefois de préférence relié à une aiguille par son insertion dans le chas de cette aiguille, et présente une extrémité libre rigidifiée pour faciliter cette insertion. Cette rigidification peut être notamment réalisée par assemblage des fils constituant le cordon au niveau de cette extrémité libre.

Chaque bouton d'arrêt peut quant à lui simplement comprendre un ou deux trous traversants, pour son engagement sur un cordon et son immobilisation par rapport à celui-ci par ligature.

A titre purement indicatif, les éléments constituant le matériel selon l'invention peuvent présenter les dimensions et caractéristiques suivantes :
- vis : 7 mm de diamètre, 24 mm de long, forme conique à son extrémité distale ;
- lien : partie tubulaire de 8 mm de longueur et de diamètre ajusté au diamètre de la vis ; cordons : tresses de fils en matériau biorésorbable ayant 1,1 mm diamètre au total et une longueur d'au moins 30 cm ;
- boutons : chacun d'eux a une épaisseur de 0,5 mm et un diamètre de 5 mm, et comprend deux trous pour sa ligature à un cordon ou une paire de cordons.

L'invention sera bien comprise, et d'autres caractéristiques et avantages de celle-ci apparaîtront, en référence au dessin schématique annexé, lequel représente, à titre d'exemple non limitatif, une forme de réalisation préférée du matériel qu'elle concerne.

La figure 1 est une vue en perspective des différents éléments composant ce matériel, et

les figures 2 à 9 sont des vues de différentes étapes successives de la réparation, au moyen de ce matériel, d'un tendon calcanéen rompu.

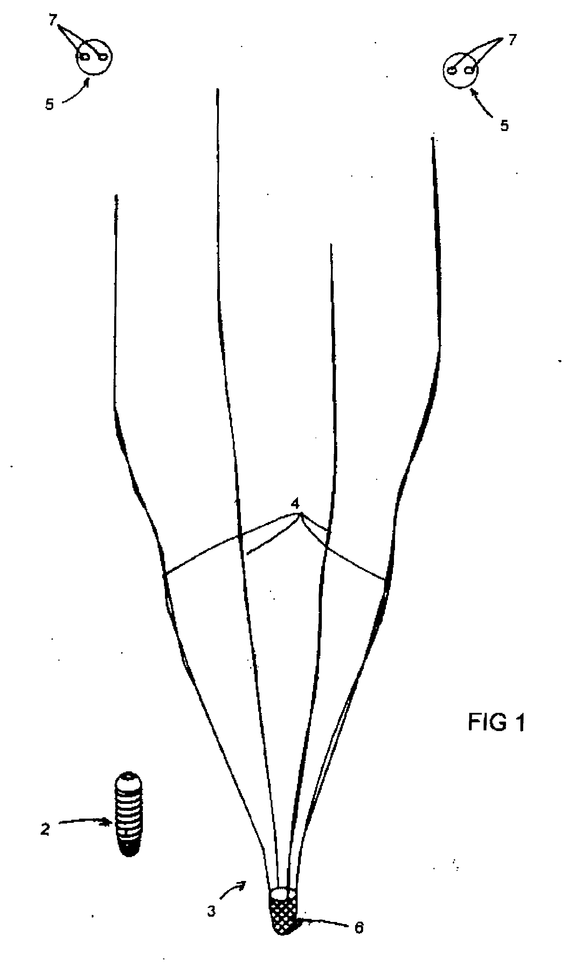

La figure 1 représente différents éléments composant un matériel de réparation d'un tissu biologique, notamment d'un tendon ou d'un ligament, et en particulier du tendon calcanéen.

Ce matériel comprend une vis osseuse 2, un lien 3 dont des parties 4 peuvent être insérées au moyen d'une aiguille au travers des fragments du tissu biologique à réparer, et deux boutons d'arrêt 5.

La vis 2 est une vis dite "d'interférence", c'est-à-dire dépourvue de tête. Elle est percée de part en part selon son axe pour son engagement sur une broche de guidage, comprend une cavité prismatique axiale permettant sa manoeuvre en rotation, et présente une forme conique à son extrémité distale, favorisant son insertion.

La vis 2 est destinée à être implantée dans un os environnant le tissu à réparer, notamment dans le calcanéum pour la réparation du tendon calcanéen, et est en matériau biorésorbable. Dans le cas de la réparation du tendon calcanéen, elle présente une longueur de 24 mm et un diamètre de 7 mm.

Le lien 3 forme une partie tubulaire 6 légèrement conique ainsi que quatre cordons 4 constituant lesdites parties pouvant être insérées au travers des fragments du tissu à réparer.

La partie tubulaire 6 est propre à recevoir étroitement la partie proximale de la vis 2 en elle. Dans le cas de la réparation du tendon calcanéen, elle a une longueur de 8 mm. Elle est réalisée par tricotage de fils en matériau biorésorbable sous la forme d'une chaussette, et les cordons 4 sont réalisés par tressage de ces mêmes fils.

Ces cordons 4 ont, dans le cas de la réparation du tendon calcanéen, 1,1 mm diamètre au total et une longueur de 35 cm.

L'extrémité libre de chaque cordon 4 est destinée à être insérée dans le chas d'une aiguille (non représentée) permettant l'introduction du cordon 4 dans les fragments du tissu à réparer, et présente une zone d'extrémité libre rigidifiée pour faciliter cette insertion. Cette rigidification est réalisée par assemblage des fils constituant le cordon 4 au niveau de cette extrémité libre.

Chaque bouton d'arrêt 5 est formé par une rondelle de matériau biorésorbable et comprend deux trous traversants 7, pour son engagement sur une paire de cordons 4 et son immobilisation par rapport à ceux-ci par ligature. Dans le cas de la réparation du tendon calcanéen, chaque bouton 5 a une épaisseur de 0,5 mm et un diamètre de 5 mm.

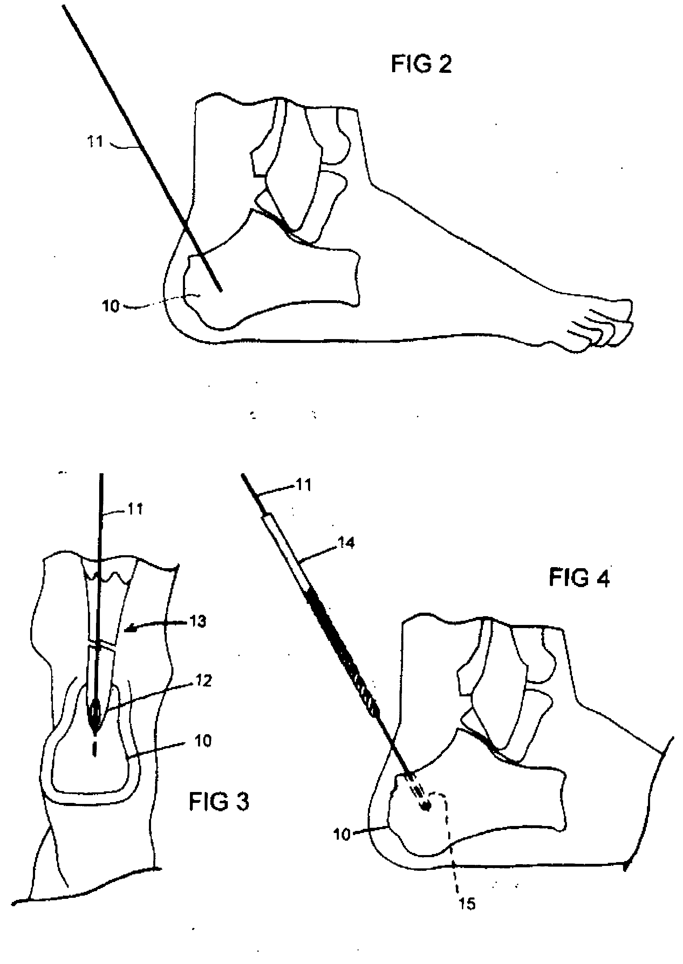

En pratique, comme le montrent les figures 2 à 4, une broche autoforante 11 est mise en place dans la calcanéum 10, en dessous de l'insertion basse 12 du tendon calcanéen 13 et au droit de celle-ci, puis une mèche canulée 14 est engagée sur cette broche 11 et est utilisée pour forer dans le calcanéum 10 un trou 15 de réception de la vis 2.

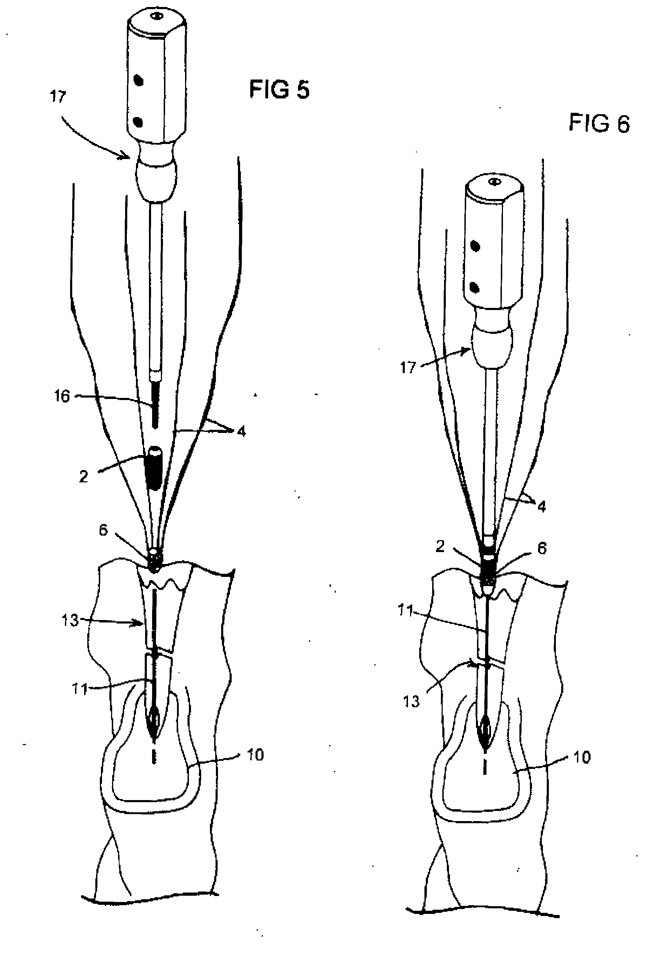

La vis 2 est engagée dans la partie tubulaire 6 de telle sorte que le tiers proximal de la vis 2 se trouve dans cette partie tubulaire 6 puis la vis 2 est engagée sur l'extrémité prismatique 16 d'un tournevis canulé 17, cette extrémité 16 étant ajustée à la cavité axiale de la vis 2, et l'ensemble est engagé sur la broche 11 (cf. figures 5 et 6).

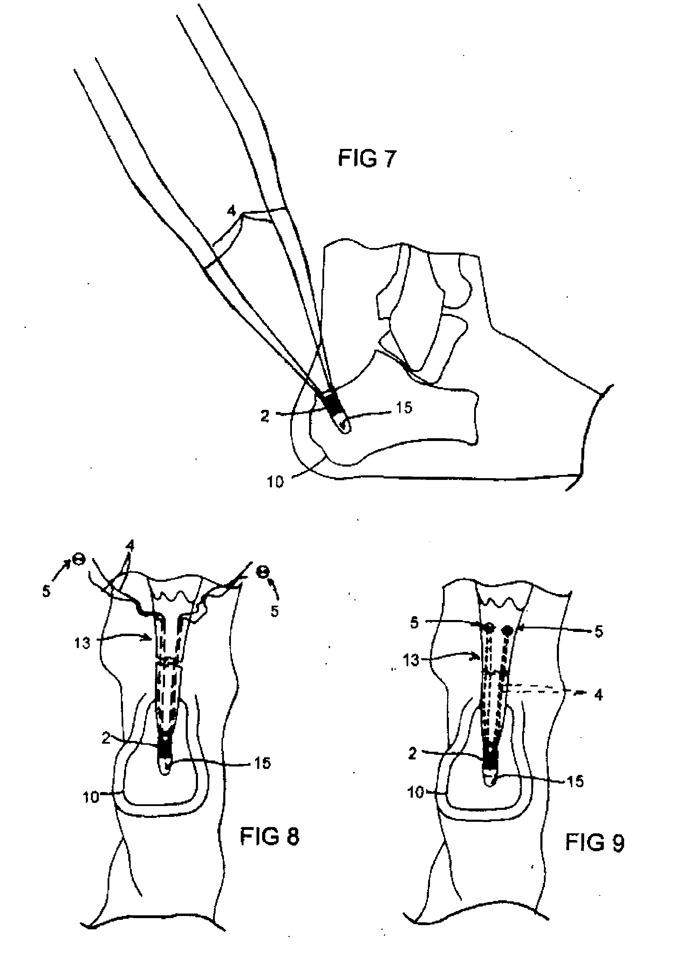

Le tournevis 17 est alors utilisé pour visser la vis 2 dans le trou 15 jusqu'à complète insertion dans ce trou de cette vis 2 et de la partie tubulaire 6, comme le montre la figure 7.

La broche 11 est ensuite retirée et les cordons 4 sont insérés dans les fragments du tendon 13, comme le montre la figure 8, puis les boutons 5 sont engagés, grâce aux trous 7 qu'ils comprennent, sur les paires de cordon 4 respectives.

Les cordons 4 sont mis en tension de manière à coapter les fragments du tendon 13, puis des ligatures des deux cordons 4 engagés au travers d'un même bouton 5 sont réalisées afin d'immobiliser le bouton 5 par rapport à ces cordons 4. Les parties de cordon 4 en excès sont ensuite sectionnées (cf. figure 9).

L'invention fournit ainsi un matériel de réparation d'un tissu biologique, notamment d'un tendon ou d'un ligament, et en particulier du tendon calcanéen, ayant pour avantage d'améliorer de manière significative les suites de l'intervention et le confort du patient en ce sens qu'il permet une rééducation plus précoce, avec, dans le cas d'un tendon calcanéen, mise en appui immédiate du pied du patient, ce qui autorise une déambulation moins périlleuse du patient. De plus, lorsque tout ou partie du matériel est biorésorbable, il devient possible de laisser le matériel en place dans l'organisme jusqu'à résorption, de sorte qu'aucune nouvelle intervention n'est nécessaire pour l'ablation du matériel et que le matériel est efficace pendant une durée, de l'ordre de trois mois, plus longue que la durée d'implantation d'un matériel selon la technique antérieure, permettant une reprise d'activité plus précoce et plus sûre pour le patient, et plus tranquilisante pour le chirurgien..

L'invention n'est pas limitée à la forme de réalisation décrite ci-dessus à titre d'exemple mais qu'elle s'étend à toutes les formes de réalisations et variantes couvertes par les revendications. Ainsi, le matériel peut être utilisé non seulement pour la réparation d'un tendon calcanéen mais également, notamment, pour la réparation de la coiffe de l'articulation de l'épaule, la réparation des ligaments croisés du genou ou la réinsertion du tendon du biceps. Dans le cas du traitement de l'articulation de l'épaule, le lien peut en particulier être en matériau non biorésorbable, la vis et les boutons d'arrêt pouvant être en matériau biorésorbable ou non biorésorbable.

## Revendications

1. - Matériel de réparation d'un tissu biologique, notamment d'un tendon ou d'un ligament, et en particulier du tendon calcanéen (13), comprenant :
- un lien (3) dont des parties (4) peuvent être insérées au travers des fragments du tissu biologique à réparer, et
- des organes (5) d'arrêt du lien (3) par rapport à ces fragments de tissu ;
matériel **caractérisé en ce que :**
- l'un des organes d'arrêt est constitué par une vis osseuse (2) pouvant être implantée dans un os environnant le tissu à réparer, notamment dans le calcanéum (10) pour la réparation du tendon calcanéen (13) ;
- le lien (3) forme une partie tubulaire (6) propre à recevoir étroitement une partie de la vis (2) en elle ainsi qu'au moins deux cordons (4) insérables au travers des fragments du tissu biologique à réparer, notamment les deux fragments d'un tendon calcanéen (13) rompu, et
- d'autres organes d'arrêt sont constitués par au moins deux boutons d'arrêt (5).

2. - Matériel selon la revendication 1, **caractérisé en ce que** la vis (2), et/ou le lien (3) et/ou les boutons d'arrêt (5) sont en matériau biorésorbable.

3. - Matériel selon la revendication 1 ou la revendication 2, **caractérisé en ce que** la vis (2) est une vis dite "d'interférence", c'est-à-dire dépourvue de tête et manoeuvrée au moyen d'une cavité prismatique débouchant dans son extrémité proximale.

4. - Matériel selon l'une des revendications 1 à 3, **caractérisé en ce que** la partie tubulaire (6) du lien (3) est réalisée par tricotage de fils sous la forme d'une chaussette, et **en ce que** les cordons (4) sont réalisés par tressage de ces mêmes fils.

5. - Matériel selon l'une des revendications 1 à 4, **caractérisé en ce que** le lien (3) comprend quatre cordons (4).

6. - Matériel selon l'une des revendications 1 à 5, **caractérisé en ce que** chaque cordon (4) présente une extrémité libre rigidifiée pour faciliter son insertion dans le chas d'une aiguille.

7. - Matériel selon l'une des revendications 1 à 6, **caractérisé en ce que** chaque bouton d'arrêt (5) comprend un ou deux trous traversants (7), pour son engagement sur un cordon (4) et son immobilisation par rapport à celui-ci par ligature.
